# EUROPEAN PATENT APPLICATION

(11) **EP 2 399 642 A1**
(43) Date of publication of application: **28.12.2011**
(21) Application number: 11167817.3
(22) Date of filing: 27.05.2011
(51) Int. Cl.: A61M 25/10

(54) **Balloon catheter**

(30) Priority: 25.06.2010 JP 2010145463
(71) Applicant: ASAHI INTECC CO., LTD., Nagoya-shi, Aichi 463-0024 (JP)
(72) Inventor: Kitagawa, Masanori, Nagoya-shi, Aichi 463-0024 (JP)
(74) Representative: Winter, Brandl, Fürniss, Hübner, Röss, Kaiser, Polte - Partnerschaft

(57) **Abstract**

An aspect of the present invention is a balloon catheter integrated with a guidewire. An object thereof is to provide a balloon catheter allowing smooth connection between a coil portion of the guidewire and a front end portion of the balloon catheter by preventing the stepped portion from being formed therebetween and allowing the tip portion of the guidewire to sufficiently exhibit characteristics thereof without providing any additional element. The balloon catheter includes: a protruding portion formed of an inner coil which is arranged inside a front end coil part and which extends from a rear end of the front end coil part to surround a core shaft; and a front end opening part of a guidewire lumen that is provided at a front end of a catheter body and into which the protruding portion is inserted.

## Description

### Cross Reference to Related Applications

This application is based on Japanese Patent Application No. 2010-145463 filed with the Japan Patent Office on June 25, 2010, the entire content of which is hereby incorporated by reference.

### Technical Field

The present invention relates to a balloon catheter used to dilate a stenosis or the like inside a body cavity such as a vessel.

### Background Art

Conventionally, a balloon catheter is used to dilate a stenosis or the like inside a body cavity such as a vessel. Various balloon catheters are known. In one example of balloon catheters, a guidewire for guiding a balloon catheter is accommodated in and non-removable from a body of the balloon catheter for the purpose of reducing the outer diameter or like purposes (see, for example, JP-A-4-261669).

In such a balloon catheter that is integrated with a guidewire, there has been a demand for making a difference in level between a coil portion provided at a tip portion of the guidewire and a front end portion of the balloon catheter as small as possible and for forming the coil portion and the front end portion of the catheter to be curved smoothly in an integrated manner.

Specifically, a stepped portion (in particular, a stepped portion formed by an outer diameter of an end of the catheter bigger than the outer diameter of the coil portion) may be formed between the coil portion of the guidewire and the front end portion of the balloon catheter. In this case, when the balloon catheter advances in a tortuous vessel, such a stepped portion may get stuck in an inner wall of the vessel or in a strut (a support forming meshes) of an indwelling stent or like disadvantages may be caused, which results in reduced crossability of the balloon catheter.

In order to prevent formation of such a stepped portion, a guidewire having an engaged portion that is raised into an arc around an outer circumference of a coil part is proposed (see, for example, JP-A-2001-204825). In this guidewire, the coil part and the front end portion of the balloon catheter are connected by the engaged portion.

### Summary of Invention

With the configuration provided with the engaged portion around the coil of the guidewire as described above, the outer diameter of the coil of the guidewire and the outer diameter of an opening part at the front end portion of the balloon catheter are increased by the amount of the outer diameter of the engaged portion. Therefore, this configuration is not necessarily desirable in terms of improving crossability of the catheter. Moreover, this configuration is provided with the additional engaged portion at the coil part of the guidewire. Therefore, it is necessary to set characteristics of the tip portion of the guidewire considering the presence of the engaged portion in addition to original characteristics of the coil part of the guidewire.

The present invention has been made in view of the above circumstances. An object of the present invention is to provide a balloon catheter integrated with a guidewire, allowing smooth connection between a coil part of the guidewire and a front end portion of the balloon catheter by preventing the stepped portion from being formed therebetween and allowing the tip portion of the guidewire to sufficiently exhibit characteristics thereof without providing any additional element.

The above object of the present invention is achieved by the means described below.

<1> A balloon catheter according to a first aspect of the present invention is a balloon catheter integrally accommodating a guidewire, including: a catheter body that includes a balloon, an inflation lumen for supplying a fluid for dilating the balloon and a guidewire lumen for accommodating the guidewire; a core shaft inserted in the guidewire lumen; a front end coil part formed of at least one wound strand and surrounding a tip portion of the core shaft extending from a front end of the catheter body; a protruding portion which is formed of at least one strand extending from a rear end of the front end coil part and wound around the core shaft; and a front end opening part of the guidewire lumen that is provided at the front end of the catheter body and into which the protruding portion is inserted.

### Brief Description of Drawings

The foregoing and other objects, features, aspects and advantages of the invention will become more apparent from the following detailed description when taken in conjunction with the accompanying drawings.
Fig. 1 is a view illustrating an entire balloon catheter according to the present embodiment.
Fig. 2 is an enlarged view illustrating a front end portion of the balloon catheter according to the present embodiment.
Fig. 3 is a cross-sectional view as seen in the direction of III-III of Fig. 1.
Fig. 4 is a cross-sectional view as seen in the direction of IV-IV of Fig. 1.
Fig. 5 is a cross-sectional view of a safety wire according to the present embodiment.
Fig. 6 is a cross-sectional view of a connector according to the present embodiment.
Fig. 7 is an explanatory drawing illustrating a function of the balloon catheter according to the present embodiment.
Fig. 8 is a view illustrating a second embodiment.

### Description of Embodiments

Preferred embodiments of the present invention will be described below with reference to the accompanying drawings, in which like reference characters designate similar or identical parts throughout the several views thereof.
<1> A first aspect of the present invention is a balloon catheter integrally accommodating a guidewire, including: a catheter body that includes a balloon, an inflation lumen for supplying a fluid for dilating the balloon and a guidewire lumen for accommodating the guidewire; a core shaft inserted in the guidewire lumen; a front end coil part formed of at least one wound strand and surrounding a tip portion of the core shaft extending from a front end of the catheter body; a protruding portion which is formed of at least one strand extending from a rear end of the front end coil part and wound around the core shaft; and a front end opening part of the guidewire lumen that is provided at the front end of the catheter body and into which the protruding portion is inserted.
<2> A second aspect of the present invention is the balloon catheter according to the first aspect, wherein the protruding portion is formed of an inner coil which is formed of at least one wound strand arranged inside the front end coil part and which extends from the rear end of the front end coil part.
<3> A third aspect of the present invention is the balloon catheter according to the second aspect, wherein a front end of the inner coil is attached to the front end of the front end coil part.
<4> A fourth aspect of the present invention is the balloon catheter according to the first aspect, wherein the protruding portion is formed of a safety wire formed of at least one strand arranged inside the front end coil part, extending from the rear end of the front end coil part and wound to surround the core shaft.

<1> The balloon catheter according to the first aspect of the present invention includes the protruding portion having a structure in which the strand is wound around the front end coil part. Furthermore, in the balloon catheter, the protruding portion is inserted into the front end opening part of the guidewire lumen of the catheter body. As a result, the front end coil part and the front end portion of the catheter body can be smoothly connected by preventing a stepped portion from being formed between the rear end portion of the front end coil part of the guidewire and the front end portion of the catheter body. Therefore, the balloon catheter can have a structure in which the front end coil part and the catheter body are curved integrally, flexibly and smoothly. Therefore, it is possible to prevent, as much as possible, a boundary portion between the front end coil part and the catheter body from getting stuck in an inner wall of a tortuous vessel, a strut of an indwelling stent or the like, and to prevent, as much as possible, reduction in crossability of the balloon catheter caused thereby or for like reasons.
   In the balloon catheter, the protruding portion is formed of a wire which extends from the front end coil part of the guidewire. It is therefore not necessary to add a special element to the front end coil part of the guidewire so as to form the protruding portion. Accordingly, setting of the front end coil part of the guidewire is facilitated. Moreover, it is possible to maximize original functions of the integrated guidewire. As a result, a guidewire with good operability can be provided.
<2> In the second aspect of the present invention, the protruding portion is formed of the inner coil provided inside the front end coil part. Therefore, the protruding portion can be easily formed.
<3> In the third aspect of the present invention, the front end of the inner coil is attached to the front end of the front end coil part. Accordingly, it is possible to make the tip portion of the core shaft surrounded by the inner coil thinner. Therefore, it is possible to reduce, as much as possible, bending of the tip portion of the core shaft by plastic deformation. Thus, the tip portion of the guidewire can be made more restorable.
   The inner coil also allows enhancement of the stiffness in the axial direction. Accordingly, it is possible to prevent lowering of the pushing force that is a force pushing the front end coil part in the axial direction even if the stiffness is lowered as a result of thinning the tip portion of the core shaft.
<4> In the fourth aspect of the present invention, the protruding portion is formed using the safety wire provided inside the front end coil part. Accordingly, it is possible to reduce, as much as possible, the influence of the formation of the protruding portion on the characteristics of the tip portion of the guidewire. In the fourth aspect, it is also possible to firmly fix the safety wire to the core shaft. As a result, in the fourth aspect, a highly safe configuration can be achieved.

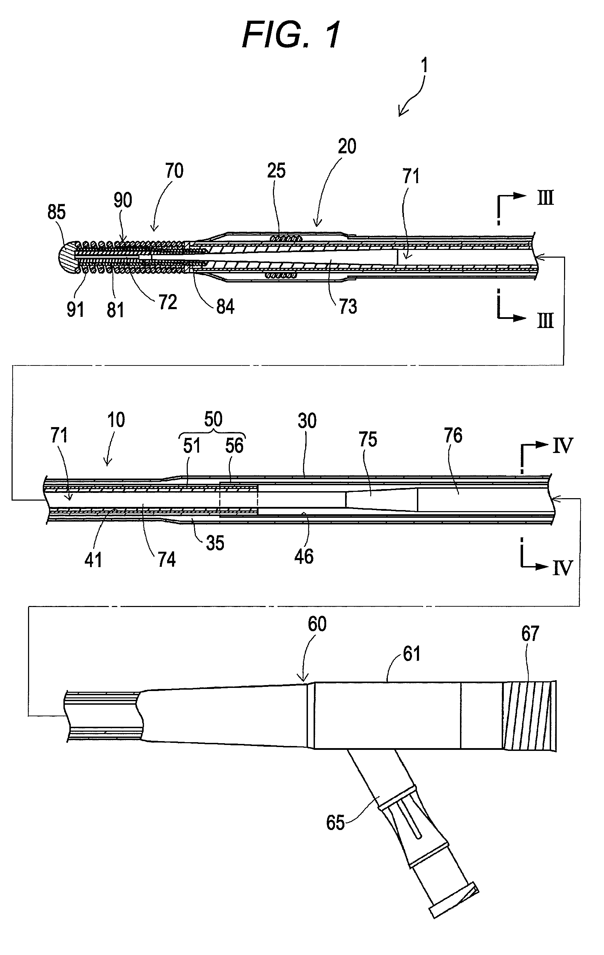

The balloon catheter according to the present embodiment will be described with reference to Figs. 1 to 6.

In Figs. 1, 2 and 6, the left side shown is the front side (distal side) to be inserted into a body, and the right side is the rear side (proximal side, base end side) to be operated by an operator such as a physician.

A balloon catheter 1 is used, for example, to treat an occlusion, a stenosis or the like of a vessel in the heart. The entire length of the balloon catheter 1 is about 1500 mm.

A guidewire 70 is accommodated in a catheter body 10 of the balloon catheter 1. The guidewire 70 is permanently integrated with the catheter body 10. The guidewire 70 is also rotatable about an axis of the catheter body 10.

The catheter body 10 mainly includes a balloon 20, an outer shaft 30, an inner shaft 50 and a connector 60.

The outer shaft 30 is a tube made of resin. A front side of the outer shaft 30 is thinner than a rear side thereof.

The outer shaft 30 has an outer diameter of about 0.50 to about 0.70 mm at the front side thereof. In the present embodiment, the outer diameter of the front side is about 0.60 mm. On the other hand, the outer shaft 30 has an outer diameter of about 0.55 to about 0.75 mm at the rear side thereof. In the present embodiment, the outer diameter of the rear side is about 0.65 mm.

Examples of the material for the resin tube used for the outer shaft 30 include resin such as polyamide, polyamide elastomer, polyolefin, polyester, and polyester elastomer.

The connector 60 is attached to a rear end of the outer shaft 30.

The inner shaft 50 is coaxially arranged inside the outer shaft 30. An inflation lumen 35 for dilating the balloon 20 is formed between the outer shaft 30 and the inner shaft 50. An indeflator (not shown) attached to the connector 60 supplies liquid for dilating the balloon 20. Then, the liquid flows through the inflation lumen 35 to dilate the balloon 20.

The inner shaft 50 includes a front side inner shaft 51 and a rear side inner shaft 56. The front side inner shaft 51 and the rear side inner shaft 56 constitute a front side guidewire lumen 41 and a rear side guidewire lumen 46, respectively.

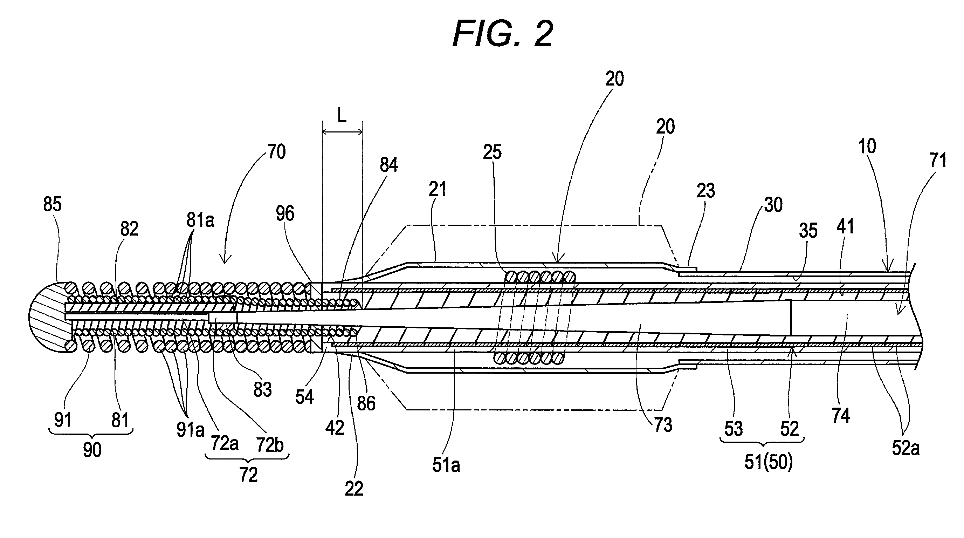

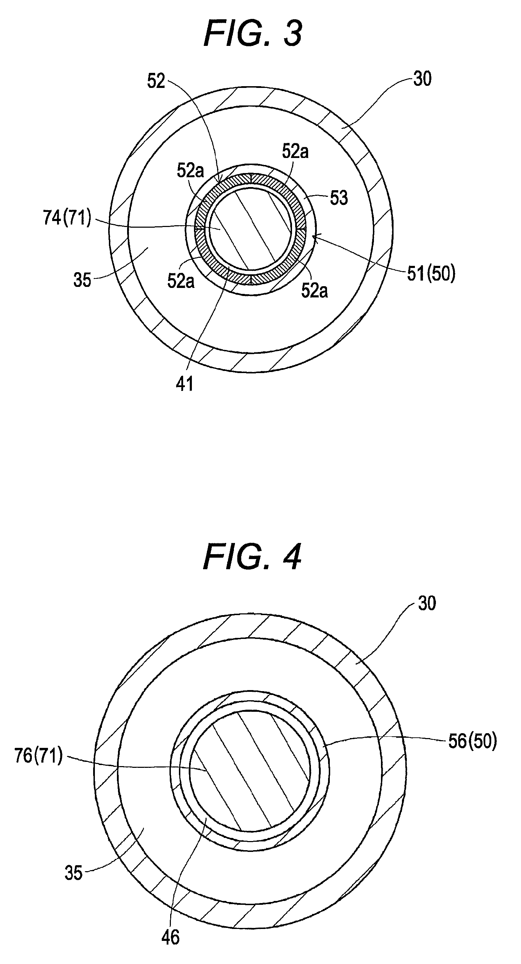

As illustrated in Figs. 2 and 3, the front side inner shaft 51 is a flexible cylindrical member having a stranded wire coil 52 and an outer resin layer 53 coated on an outer circumference of the stranded wire coil 52.

The stranded wire coil 52 is produced by winding a plurality of metal strands 52a spirally around a mandrel so as to be adjacent to each other, eliminating residual stress caused by winding using a known heat treatment, and removing the mandrel.

As illustrated in Fig. 3, four strands 52a are used for the stranded wire coil 52 according to the present embodiment. The strand 52a is a so-called flat wire having a substantially rectangular cross section. The number and size of the strand 52a are appropriately determined in view of the outer and inner diameters and stiffness required for the front side inner shaft 51. The number of the strand 52a is not limited. The strand 52a may also be a round wire having a circular cross section.

The outer resin layer 53 is formed by immersing the stranded wire coil 52 in molten resin contained in a bath and coating the resin on the outer circumference of the stranded wire coil 52. The outer resin layer 53 prevents the liquid for dilating the balloon 20 flowing through the inflation lumen 35 from leaking through gaps between the coils of strands 52a of the stranded wire coil 52 into the front side inner shaft 51.

Such a stranded wire coil 52 enables the inner shaft to have high strength while maintaining the flexibility. Thus, the balloon catheter 1 can be prevented from bending. Moreover, the balloon catheter 1 can be made thinner. Furthermore, higher pressure resistance of the balloon 20 can be set.

In the present embodiment, the front side inner shaft 51 has an outer diameter and an inner diameter of about 0.27 mm and about 0.19 mm, respectively.

Note that a heat-shrinkable resin tube may be used to make the outer resin layer 53, and the resin tube is brought into close contact with the stranded wire coil 52 by thermally shrinking the resin tube.

Examples of the material for the outer resin layer 53 includes fluorine resin and polyethylene resin in addition to resin similar to that used for the outer shaft 30 described above.

The front side inner shaft 51 has, at a front side thereof, an extension part 51a extending from a front end of the outer shaft 30. A catheter distal portion 54 made of resin is provided at a front end of the extension part 51 a. The length of the catheter distal portion 54 in the axial direction is about 0.3 mm in the present embodiment.

The material for the catheter distal portion 54 may be resin similar to that used for the outer resin layer 53 described above.

The catheter distal portion 54 is a cylindrical member that covers a front end portion of the stranded wire coil 52 and constitutes a front end portion of the front side guidewire lumen 41. A front side guidewire port 42 (front end opening part) that is an open end of the front side guidewire lumen 41 is formed at a front end of the catheter distal portion 54. The catheter distal portion 54 has an outer diameter of about 0.27 mm. The front side guidewire port 42 has an inner diameter of about 0.19 mm.

The catheter distal portion 54 can be formed integrally with the outer resin layer 53 by covering a front endface of the stranded wire coil 52 with resin forming the outer resin layer 53 in formation of the outer resin layer 53 by immersing the stranded wire coil 52 in the resin contained in the bath as described above.

Alternatively, the catheter distal portion 54 may be a separate member from the outer resin layer 53.

The balloon 20 is a member made of resin. The balloon 20 has a dilation part 21 for dilating the balloon 20 at the center in the axial direction thereof. The balloon 20 also has a front end attachment part 22 and a rear end attachment part 23 at the front and rear sides thereof, respectively. The front end attachment part 22 is firmly attached to the tip portion of the extension part 51a of the inner shaft 50 with the catheter distal portion 54 protruding out. The rear end attachment part 23 is attached to the front end of the outer shaft 30. In the present embodiment, the rear end attachment part 23 is firmly attached to the outer circumferential surface of the front end of the outer shaft 30.

Note that solid lines in Figs. 1 and 2 illustrate a state where the balloon 20 is folded before use. Alternate long and two short dashes lines in Fig. 2 illustrate a state where the balloon 20 is dilated.

A marker 25 is attached at the center inside the dilation part 21 of the balloon 20 at the extension part 51 a of the front side inner shaft 51. The marker 25 is constituted by a coil formed by winding one strand made of a radiopaque alloy including platinum and the like.

The rear side inner shaft 56 is a tubular member made of metal, a so-called hypotube, having therein the rear side guidewire lumen 46. A rear end portion of the front side inner shaft 51 is inserted into, and firmly attached to, a tip portion of the rear side inner shaft 56. As a result, the rear side guidewire lumen 46 is in communication with the front side guidewire lumen 41.

In the present embodiment, the rear side inner shaft 56 has an outer diameter of about 0.33 mm and an inner diameter of about 0.29 mm. The material for the rear side inner shaft 56 is not particularly limited, but stainless steel is used as the material in the present embodiment. Other materials that may be used include a super elastic alloy such as a Ni-Ti alloy. A resin tube may also be used.

A rear end portion of the rear side inner shaft 56 is attached to the connector 60.

Next, the guidewire 70 accommodated in the inner shaft 50 will be described. The guidewire 70 mainly includes a core shaft 71 and a front end coil part 90. The front end coil part 90 includes an inner coil 81 and an outer coil 91.

The core shaft 71 is a member having a circular cross-section and becomes thinner toward a front end thereof so that the core shaft 71 is more flexible toward the front end. The core shaft 71 includes a most distal portion 72, a first tapered portion 73, a first cylindrical portion 74, a second tapered portion 75 and a second cylindrical portion 76 in this order from the front end thereof. The first cylindrical portion 74 and the second cylindrical portion 76 each have a constant diameter. On the other hand, the first tapered portion 73 and the second tapered portion 75 each have an outer diameter gradually decreasing so as to become thinner toward the front end.

The front portion of the core shaft 71 from the thinner first cylindrical portion 74 is mainly accommodated in the front side inner shaft 51. The second cylindrical portion 76 and the second tapered portion 75 that have the largest diameter are accommodated in the rear side inner shaft 56. Predetermined gaps are formed between an outer circumferential surface of the first cylindrical portion 74 and an inner circumferential surface of the front side inner shaft 51 and between an outer circumferential surface of the second cylindrical portion 76 and an inner circumferential surface of the rear side inner shaft 56, respectively. These gaps allow the guidewire 70 to turn around the axis inside the inner shaft 50.

The material for the core shaft 71 is not particularly limited, but stainless steel (SUS304) is used as the material for the core shaft 71 in the present embodiment. Other materials that may be used include a super elastic alloy such as a Ni-Ti alloy, a piano wire and the like.

Note that additional tapered portions or cylindrical portions may be provided as necessary between the tapered portions 73 and 75 and the cylindrical portions 74 and 76. The angle and the size of the tapered portions can also be appropriately set as necessary.

The most distal portion 72 of the core shaft 71 is arranged inside the inner coil 81 that will be described later. The most distal portion 72 of the core shaft 71 includes a first flexible portion 72a at the front side and a second flexible portion 72b at the rear side. The most distal portion 72 is made as thin as possible so that a restorability thereof is enhanced. Specifically, the most distal portion 72 has such characteristic that even if the front end of the guidewire 70 is under a load during an operation, the front end is restored without bending by plastic deformation. In addition, the most distal portion 72 is to be subjected to a process called shaping. Shaping is a process of intentionally bending the front end of the guidewire 70 in a desired direction in advance by an operator such as a physician.

The first flexible portion 72a and the second flexible portion 72b each have a circular cross-section with a constant diameter. The first flexible portion 72a has a smaller diameter than the second flexible portion 72b. Thus, there is a very small tapered portion (not shown) between the first flexible portion 72a and the second flexible portion 72b.

Note that the first flexible portion 72a may be formed by press working into a flat portion having a substantially rectangular cross section.

The inner coil 81 is attached to surround the most distal portion 72 and the tip portion of the first tapered portion 73 of the core shaft 71. The stranded wire coil constituting the inner coil 81 has both stiffness and flexibility. Thus, the inner coil 81 allows the most distal portion 72 to be thinner without deteriorating the flexibility and the stiffness. As a result, the restorability of the most distal portion 72 as described above can be enhanced. The inner coil 81 is also stiff in the axial direction. It is thus possible to prevent a decrease in the transmissibility of the pushing force that is a force of the guidewire 70 pushing in the axial direction even if the most distal portion 72 becomes so thin that the stiffness thereof is lowered.

The inner coil 81 is a hollow stranded wire coil produced by winding a plurality of metal strands 81a spirally around a mandrel so as to be adjacent to each other, eliminating residual stress caused by winding using a known heat treatment, and removing the mandrel. The inner coil 81 has an outer diameter of about 0.17 mm in the present embodiment. In addition, the inner coil 81 has a length in the axial direction of about 33.0 mm.

Six strands 81a are used for the inner coil 81. The strands 81a each have a diameter of about 0.03 mm. The number and the diameter of the strands 81a are appropriately determined in view of the outer diameter and the stiffness required for the inner coil 81, and are not limited to these values.

The material for the strand 81 a is not particularly limited, but stainless steel is used as the material for the strand 81a in the present embodiment. Other materials that may be used include a super elastic alloy such as a Ni-Ti alloy. Furthermore, the inner coil 81 may be formed of a combination of strands made of different materials.

A front end of the inner coil 81 is bonded to a front end of the core shaft 71 together with a front end of the outer coil 91 by brazing around the axis of the core shaft 71. The brazed portion forms a substantially semispherical coil tip 85 (front end joint). A rear end of the inner coil 81 is bonded to the first tapered portion 73 by brazing. The brazed portion forms an inner rear end joint 86.

A portion for a length L from the rear end of the inner coil 81 constitutes a protruding portion 84 protruding from the outer coil 91. The length L is preferably at least 1.0 mm or longer. In the present embodiment, the length L is about 3.0 mm. The protruding portion 84 is inserted into the front side inner shaft 51 through the front side guidewire port 42 of the catheter distal portion 54. A very small gap that allows the protruding portion 84 to turn is formed between the protruding portion 84 and the front side inner shaft 51.

Note that the gap between the protruding portion 84 and the front side guidewire port 42 is shown in a somewhat exaggerated manner in Fig. 2 so as to illustrate the structure of the protruding portion 84.

A safety wire 82 is attached between the coil tip 85 and the second flexible portion 72b in the inner coil 81 and substantially parallel to the most distal portion 72 of the core shaft 71. The safety wire 82 is to prevent the most distal portion 72 or the like of the guidewire 70 from being separated when the most distal portion 72 or the like is overloaded inside a body.

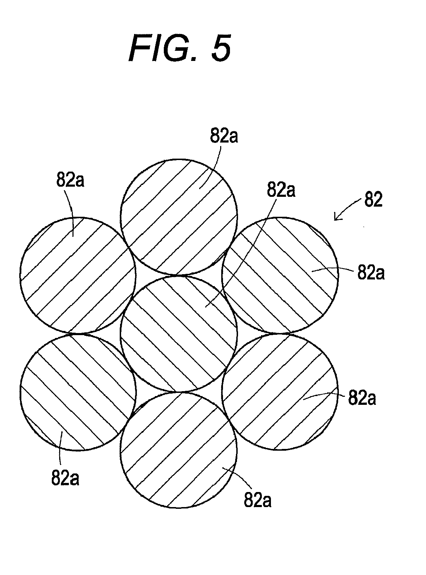

The safety wire 82 is a stranded wire produced by twisting a plurality of metal strands 82a together as illustrated in Fig. 5. The safety wire 82 has an outer diameter of about 0.042 mm in the present embodiment. Seven strands 82a are used for the safety wire 82, but the number of strand is not limited.

It is advantageous to use such a stranded wire as the safety wire 82 in terms of producing a safety wire that is flexible and less likely to break. However, a single wire or a flat wire having a substantially rectangular cross section may alternatively be used for the safety wire 82.

The material for the strand 82a is not particularly limited, but stainless steel is used as the material for the strand 82a in the present embodiment. Furthermore, the safety wire 82 may be formed of a combination of the strands made of different materials.

A front end of the safety wire 82 is bonded to the front end of the core shaft 71 at the coil tip 85 together with the inner coil 81 and the outer coil 91 by brazing. A rear end of the safety wire 82 is bonded to the second flexible portion 72b together with the inner coil 81 by brazing, which thereby forms an inner intermediate joint 83. Note that the inner intermediate joint 83 is not bonded to the outer coil 91.

The outer coil 91 surrounds a portion of the inner coil 81 other than the protruding portion 84 that is a rear end portion of the inner coil 81. The outer coil 91 is formed by winding one strand 91a made of metal. The outer coil 91 has an outer diameter of about 0.30 mm in the present embodiment. The outer diameter of the outer coil 91 is set to be slightly larger than that of the catheter distal portion 54.

The strand 91 a of the outer coil 91 is formed of a radiopaque metal wire such as a platinum alloy. Alternatively, a single strand formed by bonding a radiopaque metal wire to a radiolucent metal wire such as stainless steel may be used as the strand 91a. The strand 91a has an outer diameter of about 0.055 mm in the present embodiment.

At a front side of the outer coil 91, the strand 91 a is open coiled in a manner that gaps are formed between turns of the strand 91 a so as to increase flexibility. At a rear side of the outer coil 91, on the other hand, the strand 91 a is close coiled in a manner that adjacent turns of the strand 91a are substantially in contact with each other.

The front end of the outer coil 91 is bonded to the front end of the core shaft 71 coaxially with the inner coil 81 at the coil tip 85 by brazing. A rear end of the outer coil 91 is bonded to a side face of the inner coil 81 by brazing. The brazed portion forms an outer rear end joint 96. The bonding position of the outer rear end joint 96 is set so that the rear end portion of the inner coil 81 protrudes rearward for the length L to form the protruding portion 84.

The outer rear end joint 96 of the outer coil 91 is in contact with a front endface of the catheter distal portion 54 and rotatable relative to the front endface of the catheter distal portion 54. The outer diameter of the outer coil 91 herein is set to be slightly larger than that of the catheter distal portion 54. As a result, the endface of the catheter distal portion 54 is prevented from coming in contact with an inner wall of a vessel, a strut of an indwelling stent or the like and getting stuck therein even if a curve is formed between the outer rear end joint 96 and the catheter distal portion 54. Thus, the advancing of the balloon catheter 1 in a vessel is prevented from being blocked and the endface of the catheter distal portion 54 is prevented from damaging inside of a body.

A rear end portion of the guidewire 70 is attached to the connector 60.

The connector 60 is a member having a substantially Y-shape, and mainly includes a body part 61, a fluid port part 65 and an operating part 67.

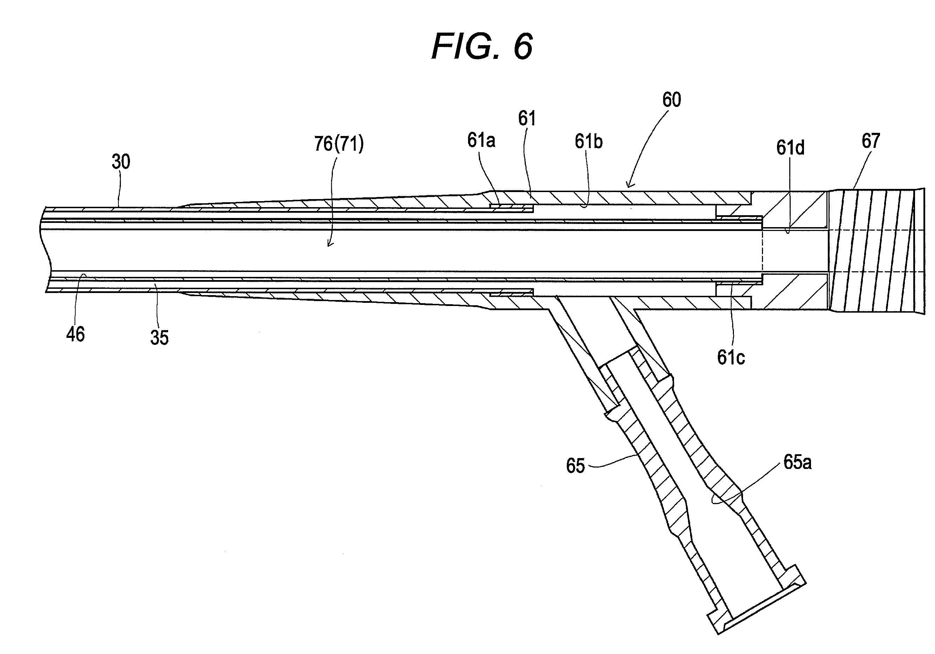

As illustrated in Fig. 6, the body part 61 has therein a front end fixing portion 61a, a fluid chamber 61b, a rear end fixing portion 61c and a rear side guidewire port 61d that are formed coaxially in this order from the front side.

The rear end portions of the outer shaft 30 and the inner shaft 50 are inserted through the front end fixing portion 61 a. The front end fixing portion 61 a is a portion that liquid-tightly fixes the rear end of the outer shaft 30 to the connector 60.

The fluid chamber 61b is connected to the inflation lumen 35. The fluid chamber 61b is also a portion into which the liquid for dilating the balloon 20 supplied through the fluid port part 65 flows.

The rear end fixing portion 61 c is a portion that liquid-tightly fixes the rear end of the inner shaft 50, which extends from the rear end of the outer shaft 30 through the fluid chamber 61b, to the connector 60.

The rear side guidewire port 61 d is a portion through which the rear end portion of the guidewire 70 extends from the rear end of the inner shaft 50. The rear end of the guidewire 70 is firmly attached to the operating part 67.

The fluid port part 65 is a portion branching and extending from the body part 61 and has a fluid supply passage 65a. One end of the fluid supply passage 65a communicates with the fluid chamber 61b. The indeflator (not shown) is connected to the other end of the fluid supply passage 65a. With this configuration, the liquid, such as a contrast agent or saline, for dilating the balloon 20 is supplied from the indeflator into the connector 60, and then supplied to the balloon 20 through the inflation lumen 35. The balloon 20 is thus dilated.

The operating part 67 is for operating the guidewire 70. An operator such as a physician can turn the guidewire 70 by turning the operating part 67 around an axis of the body part 61.

A case of using the balloon catheter 1 according to the present embodiment in an operation of dilating a stenosis in a coronary artery of the heart will be described based on the above configuration.

The balloon catheter 1 is inserted into the coronary artery of the heart where the stenosis as a target to be treated is located. The balloon catheter 1 integrally accommodates the guidewire 70. Thus, the balloon catheter 1 can be inserted into a vessel without inserting a guidewire into the vessel in advance. In the balloon catheter integrated with the guidewire, the gap between the guidewire lumen and the guidewire can be made as small as possible as compared to a common balloon catheter into which a guidewire can be removably inserted. It is thus possible to reduce the outer diameter of the balloon catheter. As a result, the balloon catheter 1 according to the present embodiment can be made to advance into a vessel with relative ease.

In particular, when the balloon catheter according to the present embodiment is used for a technique (the so-called kissing balloon technique) in which a plurality of balloon catheters is used at the same time, the balloon catheters can be inserted easily since the diameter thereof is reduced. Moreover, since the guidewire is accommodated inside the catheter, it is possible to effectively prevent the guidewire and the catheter from getting tangled with each other.

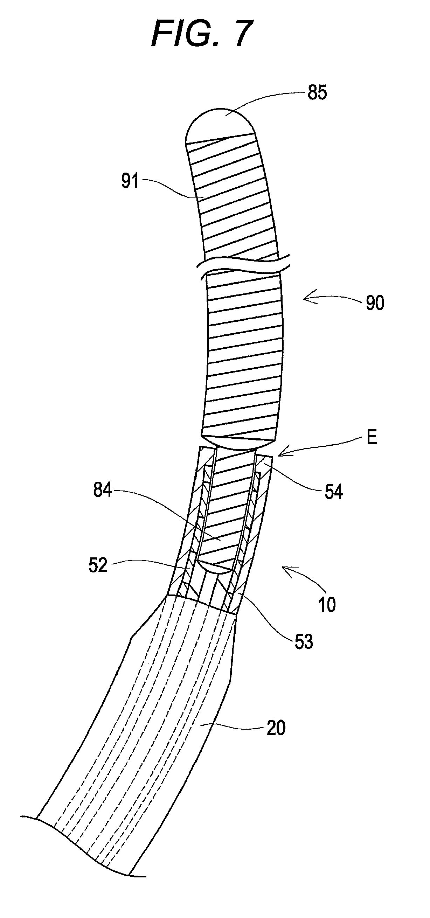

While the balloon catheter 1 is advanced into a vessel, the catheter body 10 and the guidewire 70 advance integrally. In the balloon catheter 1, the protruding portion 84 of the inner coil 81 of the guidewire 70 is inserted into the inner shaft 50 through the catheter distal portion 54. As a result, it is possible to prevent, as much as possible, a stepped portion from being formed between the outer coil 91 of the guidewire 70 and the catheter distal portion 54 even when the balloon catheter 1 passes through a tortuous vessel or the like. Therefore, the balloon catheter 1 is curved smoothly as illustrated in Fig. 7. Specifically, the protruding portion 84 has a flexible structure formed of a stranded wire coil. The protruding portion 84 is thus curved flexibly under an external force applied to the boundary between the front end coil part 90 of the guidewire 70 and the catheter distal portion 54. As a result, the portion between the outer coil 91 and the catheter distal portion 54 is curved smoothly.

In addition, the outer diameter of the outer coil 91 is set to be slightly larger than that of the catheter distal portion 54. As a result, it is possible to prevent, as much as possible, a corner E of the front endface of the catheter distal portion 54 from getting stuck in an inner wall of a vessel, a strut of an indwelling stent or the like. Note that the neighboring part of the corner E is shown in an exaggerated manner in Fig. 7 for easier understanding.

Moreover, the protruding portion 84 is formed using the rear end portion of the inner coil 81 that is included as a fundamental element in the front end coil part 90 of the guidewire 70. It is therefore not necessary to add a special element to the front end coil part 90 of the guidewire 70 so as to form the protruding portion 84. Therefore, the guidewire 70 can fully exhibit its original characteristics.

If an operator such as a physician intends to turn the front end coil part 90 of the guidewire 70 while advancing the balloon catheter 1, the operator turns the operating part 67 of the connector 60. The turning of the guidewire 70 from the proximal side rotates the front end coil part 90 integrally (that is, the outer coil 91 and the inner coil 81 simultaneously) via the core shaft 71.

Such turning of the front end coil part 90 is performed in a case where the tip portion of the guidewire 70 comes in contact with a tortuous vessel wall and the advance of the balloon catheter 1 is thus blocked, or in like cases. Such turning is also performed, in a case where the most distal portion 72 of the core shaft 71 is provided with a shaping and oriented, so as to turn the shaped direction to an appropriate direction.

The operator positions the balloon 20 at the stenosis as a target site, using the marker 25 under radioscopy. Subsequently, the liquid for dilation such as a contrast agent or saline is supplied by the indeflator (not shown) connected to the fluid port part 65 of the connector 60.

At this time, the liquid for dilation flows into the inflation lumen 35 formed between the outer shaft 30 and the inner shaft 50 through the fluid chamber 61b of the body part 61 of the connector 60, and dilates the balloon 20.

Upon completion of the operation of dilating the stenosis with the balloon 20, the operator uses the indeflator to discharge the liquid for dilation out of the balloon 20. That is, the liquid for dilation is discharged from inside the balloon 20 to the indeflator through the inflation lumen 35.

As described above, the balloon catheter 1 according to the present embodiment has a structure integrally including the guidewire 70. Moreover, in the balloon catheter 1, the protruding portion 84 that is a rear end portion of the inner coil 81 of the guidewire 70 is inserted into the catheter body 10. Accordingly, a stepped portion is substantially not formed between the rear end portion of the front end coil part 90 of the guidewire 70 and the front end portion of the catheter body 10. In other words, a smooth connection is provided at the boundary between the front end coil part 90 and the catheter distal portion 54. The balloon catheter 1 is thus flexibly and smoothly curved as a whole. Therefore, it is possible to prevent, as much as possible, the endface of the catheter distal portion 54, which is a front end portion of the catheter body 10, from getting stuck in an inner wall of a vessel, a strut of an indwelling stent or the like inside a tortuous vessel, and to prevent, as much as possible, reduction in crossability of the balloon catheter 1 caused thereby or for like reasons.

The protruding portion 84 is formed by the inner coil 81 of the guidewire 70. It is therefore not necessary to add a special element to the front end coil part of the guidewire 70. As a result, it is possible to prevent, as much as possible, delicate operations of the guidewire 70 from being blocked.

The safety wire 82 according to the present embodiment described above has an advantage that it is possible to prevent the tip portion of the core shaft 71 from bending, as described above. However, if the core shaft 71 is sufficiently stiff or if another safety measure is taken, the safety wire 82 is not absolutely necessary.

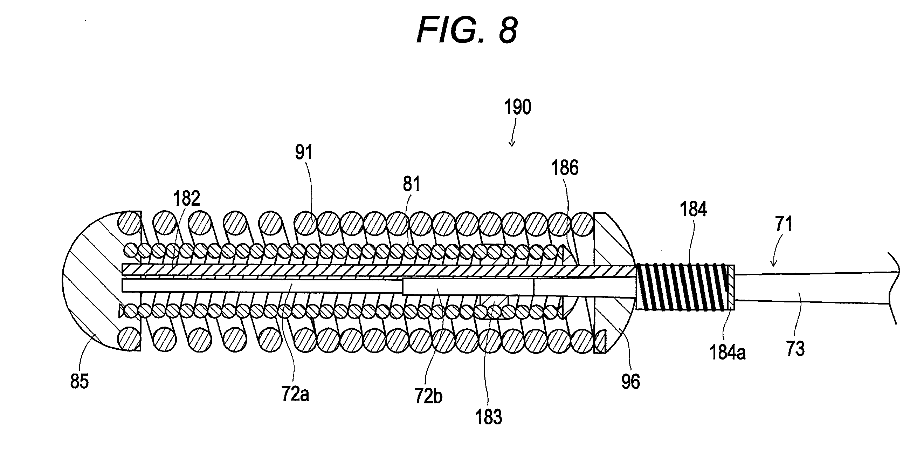

Fig. 8 illustrates a protruding portion of a front end coil part according to the second embodiment. In the embodiment described above, the protruding portion 84 is formed by extending the inner coil 81 from the rear end of the outer coil 91. In the second embodiment, a protruding portion 184 is formed by extending a safety wire 182 from the rear end of the outer coil 91 and winding the extended safety wire 182 around the first tapered portion 73 of the core shaft 71. A rear end of the protruding portion 184 is bonded to the first tapered portion 73 of the core shaft 71 by brazing. The brazed portion forms a rear end joint 184a.

With such a configuration, an inner rear end joint 186 of the inner coil 81 is located inside the outer coil 91. In addition, the rear side of the safety wire 182 is bonded at a plurality of joints including an inner intermediate joint 183 that is a joint with the second flexible portion 72b of the core shaft 71, the inner rear end joint 186 of the inner coil 81, the outer rear end joint 96 of the outer coil 91, and the rear end joint 184a of the protruding portion 184. It is thus possible to firmly fix the safety wire 182 to the core shaft 71. As a result, the safety of the safety wire 182 can be increased.

The safety wire 182 is a member additionally attached so as to increase the safety. Thus, the safety wire 182 has little impact on the tip portion of the guidewire. It is possible to reduce, as much as possible, the influence of the formation of the protruding portion 184 on the characteristics of the tip portion of the guidewire by forming the protruding portion 184 using the safety wire 182.

The safety wire 182 is preferably a stranded wire formed by twisting a plurality of strands together similarly to the safety wire 82 described above. This is advantageous in flexibility, easy winding around the core shaft 71 and the like. However, a single wire or a wire rod having a substantially rectangular cross section may alternatively be used for the safety wire 182.

Note that a front end coil part 190 according to the second embodiment has a double coil structure including the inner coil 81 and the outer coil 91. However, the inner coil 81 is not absolutely necessary. It is, however, effective to provide the inner coil 81 because the restorability of the tip portion of the core shaft 71 can be enhanced by making the tip portion of the core shaft 71 thinner as described in the first embodiment.

In the embodiments described above, the guidewire 70 is only allowed to turn around the axis of the catheter body 10. However, the guidewire 70 may be capable of moving along the axial direction for a predetermined length. In such case, the rear end of the core shaft 71 may extend from the body part 61 of the connector 60. With this configuration, both the movement of turning around the axis and the movement along the axial direction can be achieved by operating the operating part 67.

In the embodiments described above, the inner coil 81 and the stranded wire coil 52 of the front side inner shaft 51 are stranded wire coils made of a plurality of strands. However, these may be single wire coils each formed of one strand. However, a stranded wire coil is to allow maintenance of flexibility and increase in stiffness. Thus, the inner coil 81 and the stranded wire coil 52 are preferably formed using stranded wire coils each made of a plurality of strands as in the embodiments described above. As a result, it is possible to improve, as much as possible, torque transmissibility for transmitting a force pushing the guidewire 70 or the catheter body 10 in the axial direction or transmitting a force in the rotational direction of the guidewire 70.

The inside of the stranded wire coil 52 may be coated with polyamide resin, fluorine resin, polyethylene resin or the like. More preferably, low-friction resin is used for the coating.

On the other hand, it is effective that the front side inner shaft 51 includes the stranded wire coil 52 in terms of transmitting the force pushing the catheter body 10 in the axial direction and increasing the stiffness of the front side inner shaft 51. However, the front side inner shaft 51 may alternatively be made only of resin.

In the embodiments described above, the balloon catheter 1 is used for treatment of a vessel in the heart. However, the balloon catheter 1 is not limited thereto, and may be used for various operations including an operation of dilating a vessel in the lower limb or a dialysis shunt.

While the invention has been shown and described in detail, the foregoing description is in all aspects illustrative and not restrictive. It is therefore understood that numerous modifications and variations can be devised without departing from the spirit and scope of the invention.

## Claims

**1.** A balloon catheter (1) integrally accommodating a guidewire (70), comprising:
a catheter body (10) that includes a balloon (20), an inflation lumen (35) for supplying a fluid for dilating the balloon (20) and a guidewire lumen (41, 46) for accommodating the guidewire (70);
a core shaft (71) inserted in the guidewire lumen (41, 46);
a front end coil part (90, 190) formed of at least one wound strand and surrounding a tip portion of the core shaft (71) extending from a front end of the catheter body (10);
a protruding portion (84, 184) which is formed of at least one strand extending from a rear end of the front end coil part (90, 190) and wound around the core shaft (71); and
a front end opening part (42) of the guidewire lumen (41, 46) that is provided at the front end of the catheter body (10) and into which the protruding portion (84, 184) is inserted.

**2.** The balloon catheter (1) according to claim 1, wherein the protruding portion (84) is formed of an inner coil (81) which is formed of at least one wound strand arranged inside the front end coil part (90) and which extends from the rear end of the front end coil part (90).

**3.** The balloon catheter (1) according to claim 2, wherein a front end of the inner coil (81) is attached to the front end of the front end coil part (90).

**4.** The balloon catheter (1) according to claim 1, wherein the protruding portion (184) is formed of a safety wire (182) formed of at least one strand arranged inside the front end coil part (190), extending from the rear end of the front end coil part (190) and wound to surround the core shaft (71).
